# EUROPEAN PATENT SPECIFICATION

(11) **EP 1 365 810 B1**
(45) Date of publication and mention of the grant of the patent: **06.01.2010**
(21) Application number: 02717476.2
(22) Date of filing: 14.02.2002
(51) Int. Cl.: A61K 47/00, A61K 31/44, A61K 33/10

(54) **CALCIUM SUPPLEMENTATION TO REDUCE PROSTATE CANCER RISK**
CALCIUM-ERGÄNZUNG ZUR SENKUNG DES RISIKOS FÜR PROSTATAKREBS
APPORT COMPLEMENTAIRE DE CALCIUM REDUISANT LE RISQUE DE CANCER DE LA PROSTATE

(30) Priority: 16.02.2001 US 269256 P
(43) Date of publication of application: 03.12.2003
(73) Proprietor: Trustees of Dartmouth College, Hanover, NH 03755-1404 (US)
(72) Inventor: BARON, John A., Dartmouth College, Lebanon, NH 03766 (US)
(74) Representative: Howard, Paul Nicholas
(86) International application number: PCT/US2002/005214
(87) International publication number: WO 2002/066065

(56) References cited:
- US-A- 5 886 012
- US-A- 5 886 012
- GIOVANNUCCI EDWARD ET AL: "Calcium and fructose intake in relation to risk of prostate cancer" CANCER RESEARCH, AMERICAN ASSOCIATION FOR CANCER RESEARCH, BALTIMORE, MD, US, vol. 58, no. 3, 1 February 1998 (1998-02-01), pages 442-447, XP002163897 ISSN: 0008-5472

## Description

### PRIORITY

This application claims priority under 35 U.S.C. §119 from provisional patent application serial number 60/269,256, filed February 16,2001, which is hereby incorporated by reference in its entirety.

The research leading to the present invention was supported, in part, by National Institutes of Health Grant No. CA37287. Accordingly, the Government may have certain rights in the invention.

### FIELD OF THE INVENTION

The present invention relates to promoting prostate health by reducing the risk, and optimally preventing, carcinogenesis of the prostate through modification of oral intake. In particular, ingestion of calcium supplements results in reduced risk of prostate cancer.

### BACKGROUND OF THE INVENTION

Dietary patterns have repeatedly been associated with the risk of colorectal neoplasia: a diet rich in vegetables and fruits is associated with a lower risk, while intake of animal fat and red meat seems to increase risk (Sandler, Gastroenterology Clinics NA, 25:717-735, 1996). The underlying mechanisms are not clear, but may in part be due to alterations in bile acids, which are carcinogenic in animal models (Nagengast et al., Eur. J. Cancer, 1995, 31A:1067-70).

Newmark and colleagues (Newmark et al., J. Natl. Cancer Inst., 1984, 72:1323-1325) proposed that calcium binds bile acids in the bowel lumen, inhibiting their proliferative and carcinogenic effects. In support of this hypothesis, animal studies have indicated a protective effect of dietary calcium on bile-induced mucosal damage and experimental bowel carcinogenesis (Pence, Mut. Res., 1993, 290:87-95; Pence, Carcinogenesis, 1988, 9:187-190). However, human epidemiological research has been inconsistent; in some studies a decreased risk of colorectal cancer has been associated with calcium intake, while in others, no association was found (Blergsma-Kadijk et al., Epidemiology, 1996, 7:590-597; Martinez and Willett, Cancer Epidemiol. Biomarkers Prev., 1998, 7:163-168). Similarly mixed results have been reported regarding large bowel adenomas, likely precursors for most colorectal cancers (Morson et al., Cancer Surv., 1983, 2:451-477).

The protective effect of calcium supplementation in preventing colorectal carcinoma may be due, in part, to unique features of the human digestive system, binding of bile acids, or to some other mechanism. None of these explanations provide any indication that calcium supplements, taken orally, affect the development or course of any other cancers. Indeed, given the apparent relationship between oral calcium supplements and cancer of the lower bowel, no such associations can be expected.

Recently, Baron described that 1200 mg of elemental calcium (supplied in 3000 mg of calcium carbonate) administered once or twice daily resulted in decreased risk of recurrent colorectal adenomas in patients with a history of colorectal adenomas (U.S. Patent No. 6,251,439). In particular, 930 patients with a recent history of colorectal adenomas were randomized to calcium carbonate (3 gm daily; 1,200 mg elemental calcium or placebo), with follow-up colonoscopies one and four years after the qualifying examination. The main analysis focused on new adenomas found after the first follow-up endoscopy, up to (and including) the second follow-up examination. Risk ratios of at least one recurrent adenoma and ratios of the • average numbers of adenomas and 95% confidence intervals were calculated as measures of effect.

As a result of this treatment protocol, there was a lower risk of recurrent adenomas in subjects randomized to calcium. Among the 913 subjects who had at least one study examination, the adjusted risk ratio of any adenoma recurrence was 0.85 (95% confidence interval 0.74 to 0.98; P = 0.04); the adjusted ratio of the average numbers of adenomas was 0.76 (95% confidence inerval 0.60 to 0.96; P = 0.02). The effect of calcium was independent of initial dietary fat and calcium intake. No toxicity was associated with supplementation. These findings indicate that calcium supplementation can prevent a proportion of colorectal adenomas, precursors of most colorectal cancers. Thus, this work resolved the uncertainty with reject to the effects of elemental calcium on colorectal cancer: calcium has a protective effect in this form of cancer.

US 5 886 012 discloses that a combination therapy of a calcium channel blocker and a calcium supplement is useful in the treatment of parathyroid hypertersine factor mediated diseases.

An analysis of the patients who received calcium supplements to determine the protective effect on colorectal cancer particularly colorectal adenomas, compared to placebo controls, has revealed a startling discovery, which forms the basis of the present invention.

### SUMMARY OF THE INVENTION

The present invention advantageously a method for preserving prostate health in a subject. Specifically discloses this invention provides the use of elemental calcium in the manufacture of a medicament to reduce the risk of prostate cancer wherein the administration of the medicament consists of administering a dose of elemental calcium to a subject in need thereof.

### DETAILED DESCRIPTION OF THE INVENTION

In one aspect, the present invention provides the use of elemental calcium in the manufacture of a medicament to reduce the risk of prostate cancer wherein the administration of the medicament consists of administering a dose of elemental calcium to a subject in need thereof. Thus the invention promotes prostate health to the advantage of patients at risk for prostate diseases or disorders, especially prostate cancer. The present invention significantly reduces the risk of developing prostate cancer after two or more years of treatment.

In a specific clinical trial, during the first two years of treatment, Kaplian-Meier curves for calcium and placebo groups were almost identical. Marked divergence of these curves occurred in the third year, with lower incidence of prostate cancer in the calcium group. These lower incidence rates manifested in a log-rank test for a full five year follow up as statistically significant, with p=0.05. The adjusted odds ratio for calcium during the four and five year follow ups were 0.54 (with a 95% confidence interval of 0.22-1.30) and 0.43 (with a 95% confidence interval of 0.20-094), respectively.

The dose of elemental calcium to be used in conjunction with the present invention can be readily established. In particular, a dose ranging from 1 mg/kg/day to 100 mg/kg/day, preferably from 1 mg/kg/day to 50 mg/kg/day, and more preferably about 20 mg/kg twice a day, can be used. In a specific embodiment, the dose of elemental calcium is 1200 to 2400 mg per day, especially about 1200 mg twice a day. In a specific embodiment, a daily dose of calcium carbonate is about 3000 mg per day, which provides a dose of about 1200 mg elemental calcium per day. Alternatively, the daily dose can be 3000 mg twice a day, which provides 2400 mg of elemental calcium.

Elemental calcium can be derived from many sources. Usually, it is found in a salt. For the purposes of the present application, elemental calcium refers to calcium as it exists in any composition (e.g., free calcium or in a calcium salt). Examples of calcium sources include, but are by no means limited to, calcium carbonate, calcium citrate, calcium hydroxide, calcium phosphate (including tricalcium phosphate and dicalcium phosphate), calcium chlorophosphate, or combinations thereof. In a specific embodiment, the calcium is provided as calcium carbonate.

When calcium carbonate is administered to the subject, a preferred dose is from 20 to 80 mg/kg twice a day, and more preferably about 40 mg/kg twice a day. In a specific embodiment, the dose is about 1500 mg or 3000 mg twice a day.

The present invention is based, in part, on data from a large, double-blind chemoprevention trial of calcium carbonate supplementation for the prevention of large bowel adenomas to determined whether calcium also may have an effect on prostate cancer. The study included 671 male participants randomized to placebo or calcium and followed for five years (from randomization to the end of treatment, plus twelve months). The analysis of these results indicates that calcium supplementation decreases the risk of prostate cancer, thus promoting prostate health. After four years of follow up, 22 men were diagnosed with prostate cancer: 8 from the calcium group and 14 from the placebo group; Chi-square p=015). After five years, 32 men were diagnosed with prostate cancer (11 calcium and 21 placebo, Chi-square p=0.05). This is the first association documented between calcium supplementation and the risk of prostate cancer in a randomized study. Randomized studies provide the most scientifically rigorous approach to testing therapeutic efficacy of a treatment regimen.

Previous studies of association between calcium supplementation and prostate cancer have been non-randomized (observational) studies, and have suggested that calcium intake either has no relationship to prostate cancer risk or increases the risk.

In a specific embodiment, the term "about" or "approximately" means within 20%, preferably within 10%, and more preferably within 5% of a given value or range.

The various elements of the invention are further elaborated in the following sections concerning carcinomas, calcium sources, formulations, and administration. These sections are provided for the sake of convenience, and are not intended to limit the scope of the invention.

### Prostate Cancer (Carcinoma)

A carcinoma is malignant new growth that arises from epithelium, found in skin or, more commonly, the lining of body organs, for example: breast, prostate, lung, stomach or bowel. Carcinomas tend to infiltrate into adjacent tissue and spread (metastasize) to distant organs, for example: to bone, liver, lung or the brain. An adenocarcinoma is a form of cancer that involves cells from the lining of the walls of many different organs of the body. Prostate cancer is a type of adenocarcinoma. Prostate cancer, the second most common malignancy in men, is a malignant tumour of glandular origin in the prostate. Over 95% are adenocarcinomas. This type of cancer is most commonly seen in older men, with the age of 73 being the average age at the time of diagnosis. A family history for prostate cancer and perhaps, a diet that is high in fat are considered to be risk factors for this malignancy. Early detection is possible through annual digital rectal examinations and routine PSA testing. Prostate carcinoma is an alternative name for prostate cancer, which has four stages, A through C. Treatment of stages A and B involves radical prostatectomy (prostate, seminal vesicles, part of bladder); some do simple prostatectomy for stage A. Treatment for stage C involves radiation therapy; stage D involves orchiectomy and/or estrogens.

A stage A tumour is discovered incidentally in tissue removed for BPH. Stage A1 involves small focal involvement of one lobe; stage A2 multifocal or diffuse carcinoma. Stage B presents with a palpable carcinoma confined to prostate on digital exam. B1 has a solitary nodule less than 1.5 cm, while B2 involves diffuse involvement of both lobes. A stage C tumour extends, through the prostate capsule with no metastasis. Stage D involves metastases, usually to bone and/or pelvic lymph nodes.

### Prostate Cancer Staging and Treatment

### Stage I Prostate Cancer

***77a, N0, M0, Well-Differentiated (Stage A1).*** The frequency of clinically silent, nonmetastatic prostate cancer that can be found at autopsy greatly increases with age, and may be as high as 50% to 60% in men aged 90 and over. Undoubtedly, the incidental-discovery of these occult cancers at prostatic surgery performed for other reasons accounts for the similar survival of men with stage I prostate cancer compared to the normal male population, adjusted for age. Many stage I cancers are well-differentiated and only focally involve the gland (T1a, N0, M0), and the majority require no treatment other than careful follow-up. In a retrospective pooled analysis, 828 men with clinically localized prostate cancer were managed by initial conservative therapy with subsequent hormone therapy given at the time of symptomatic disease progression. This study showed that the patients with grade 1 or 2 tumors experienced a disease-specific survival of 87% at 10 years and that their overall survival closely approximated the expected survival among men of similar ages in the general population. However, in younger patients (age 50-60) whose expected survival is long, treatment should be considered. Less differentiated cancers that involve more than a few pieces of resected tissue (T1b, N0, M0) are biologically more aggressive. Radical prostatectomy, external-beam radiation therapy, and interstitial implantation of radioisotopes and watchful waiting yield apparently similar survival rates in noncontrolled selected series.

### Treatment Options:

1. Careful observation without further immediate treatment in selected patients.
2. External-beam radiation therapy. Definitive radiation therapy should be delayed 4 to 6 weeks after transurethral resection to reduce incidence of stricture.
3. Radical prostatectomy usually with pelvic lymphadenectomy (with or without the nerve sparing technique designed to preserve potency). Radical prostatectomy may be difficult after a transurethral resection of the prostate. Consideration may be given to postoperative radiation therapy for patients who are found to have capsular penetration or seminal vesicle invasion by tumor at the time of prostatectomy or have a detectable level of prostate-specific antigen more than 3 weeks after surgery. Because duration of follow-up in available studies is still relatively short, the value of postoperative radiation therapy is yet to be determined. However, postoperative radiation therapy does reduce local recurrence. Careful treatment planning is necessary to avoid morbidity.
4. Interstitial implantation of radioisotopes *(i.e.,* I-125, palladium, iridium) done through a transperineal technique with either ultrasound or CT guidance is being done in carefully selected patients with T1 or T2A tumors. Short term results in these patients are similar to those for radical prostatectomy or external-beam radiation therapy. One advantage is that the implant is performed as outpatient surgery.
5. External-beam radiation therapy designed to decrease exposure of normal tissues using methods such as computed tomography-based 3-D conformal treatment planning.

### Stage II Prostate Cancer

***T2, N0, M0 (Stage A2 or B1 or B2).*** Radical prostatectomy, external-beam irradiation, and interstitial implantation of radioisotopes are each employed in the treatment of stage II prostate cancer with apparently similar therapeutic effects. Radical prostatectomy and radiation therapy yield apparently similar survival rates with up to 10 years follow-up. For well-selected patients, radical prostatectomy can achieve 15-year survival comparable to an age-matched population without prostate cancer. Unfortunately, randomized comparative trials of these treatment methods with prolonged follow-up are lacking. Patients with a small palpable cancer (T2a, N0, M0) fare better than patients in whom the disease involves both lobes of the gland (T2b, N0, M0). Patients proven free of node metastases by pelvic lymphadenectomy fare better than patients in whom this staging procedure is not performed; however, this is due to selection of patients who have a more favorable prognosis. Side effects of the various forms of therapy including impotence, incontinence, and bowel injury should be considered in determining which type of treatment to employ. The only randomized study performed to date comparing radical prostatectomy at diagnosis to expectant therapy (careful observation with therapy as needed) in stages I and II cancers did not show a significant difference in survival. In a retrospective pooled analysis, 828 men with clinically localized prostate cancer were managed by initial conservative therapy with subsequent hormone therapy given at the time of symptomatic disease progression. This study showed that the patients with grade 1 or 2 tumors experienced a disease-specific survival of 87% at 10 years and that their overall survival closely approximated the expected survival among men of similar ages in the general population. The decision to treat should be made in the context of the patient's age, associated medical illnesses, and the patient's personal desires.

The role of adjuvant hormonal therapy in patients with locally advanced disease has undergone re-evaluation. An overview analysis has been performed. Some patients with bulky T2b tumors were included in the studied groups. The meta-analysis found a difference in 5-year overall survival in favor of radiation therapy plus continued androgen suppression compared to radiation therapy alone (hazard ratio=0.631, 95% confidence interval=0.479-0.831).

### Treatment Options:

1. Radical prostatectomy usually with pelvic lymphadenectomy. If allowed by the extent of tumor, anatomical dissection that preserves nerves necessary for erection avoids impotence postoperatively in some patients. Consideration may be given to postoperative radiation therapy for patients who are found to have capsular penetration or seminal vesicle invasion by tumor at the time of prostatectomy or a detectable level of prostate-specific antigen more than 3 weeks after surgery.
2. External-beam irradiation. Prophylactic irradiation of clinically or pathologically uninvolved pelvic lymph nodes does not appear to improve overall survival or prostate cancer-specific survival. Definitive radiation therapy should be delayed 4 to 6 weeks after transurethral resection to reduce incidence of stricture. For patients with bulky T2b tumors, adjuvant hormonal therapy should be considered.
3. Careful observation without further immediate treatment (in selected patients).
4. Interstitial implantation of radioisotopes (*i.e.*, 1-125, palladium, iridium) done through a transperineal technique with either ultrasound or CT guidance is being done in carefully selected patients with T1 or T2A tumors. Short term results in these carefully selected patients are similar to those for radical prostatectomy or external-beam radiation therapy.
5. External-beam radiation therapy designed to decrease exposure of normal tissues using methods such as computed tomography based 3-D conformal treatment planning.
6. Ultrasound-guided percutaneous cryosurgery, is a surgical technique that involves destruction of prostate cancer cells by intermittent freezing of the prostate tissue with cryoprobes followed by thawing.

### Stage III Prostate Cancer

***T3, N0, M0 (Stage C).*** External-beam irradiation, interstitial implantation of radioisotopes, and radical prostatectomy are used. The results of radical prostatectomy in stage III patients are greatly inferior compared to patients with stage II cancer. Interstitial implantation of radioisotopes is technically difficult in large tumors. External-beam irradiation using a linear accelerator is the most appropriate treatment for the majority of patients with stage III prostate cancer, and large series support its success in achieving local disease control and disease-free survival. Prognosis is greatly affected by whether regional lymph nodes are evaluated and proven not to be involved. The patient's symptoms related to cancer, age, and coexisting medical illnesses should be taken into account before deciding on a therapeutic plan. In a series of 372 patients treated with radiation therapy and followed for 20 years, 47% eventually died of prostate cancer, but 44% died of intercurrent illnesses without evidence of prostate cancer.

Hormonal therapy should be considered in conjunction with radiation. Several studies have investigated its utility in patients with locally advanced disease. A prospective, randomized trial was performed by the Radiation Therapy Oncology Group (RTOG) (RTOG 85-31) in patients with T3, N0, or any T, N1, M0 disease who received prostatic and pelvic radiation therapy and then were randomized to receive immediate adjuvant goserelin or observation with administration of goserelin at time of relapse. In patients assigned to receive adjuvant goserelin, the drug was started during the last week of the radiation therapy course and was continued indefinitely or until signs of progression. The actuarial overall 5-year survival rate for the entire population of 945 analyzable patients was not statistically significantly different (75% on the adjuvant arm versus 71 % on the observation arm, p=0.52). However, improved actuarial 5-year local control rate freedom from distant metastasis and disease-free survival have been observed.

A meta-analysis found a difference in 5-year overall survival in favor of radiation therapy plus continued androgen suppression compared to radiation therapy alone (hazard ratio=0.631, 95% confidence interval=0.479-0.831). This reduction in overall mortality indicates that adjuvant androgen suppression should be initiated at the time of radiation and continued for several years. The optimal duration of therapy remains to be determined. The issue of utility of neoadjuvant hormonal therapy remains to be determined.

Additionally, a study on patients with bulky local disease (T2b, T2c, T3, or T4), with or without nodal involvement below the common iliac chain was done: 456 men were evaluable and were randomized to receive either radiation alone or radiation with androgen ablation started 8 weeks prior to radiation and continued for 16 weeks. At 5 years, overall survival was identical, and local control (54% versus 29%) and disease-free survival (36% versus 15%) favored the combined arm.(Level of evidence: 1iiA) This trial only assessed short-term hormonal therapy, not long-term therapy as the studies analyzed by the AHCPR did. Initial results from a randomized study of immediate hormonal Treatment (orchiectomy or luteinizing hormone-releasing hormone (LHRH) analogue) versus deferred treatment (watchful waiting with hormonal therapy at progression) in men with locally advanced or asymptomatic metastatic prostate cancer showed better overall survival and prostate cancer-specific survival with the immediate treatment. The incidence of pathologic fractures, spinal cord compression, and ureteric obstruction were also lower in the immediate treatment arm.

### Treatment Options:

1. External-beam radiation. Hormonal therapy should be considered in addition to external-beam radiation. Definitive radiation therapy should be delayed until 4 to 6 weeks after transurethral resection to reduce incidence of stricture. Radiation therapy is optimally designed to decrease exposure of normal tissues using methods such as computed tomography-based 3-D conformal treatment planning.
2. Hormonal manipulations (orchiectomy or LHRH agonist).
3. Radical prostatectomy usually with pelvic lymphadenectomy (highly selected patients). Consideration may be given to postoperative radiation therapy for patients who are found to have capsular penetration or seminal vesicle invasion by tumor at the time of prostatectomy or a detectable level of prostate-specific antigen more than 3 weeks after surgery.
4. Careful observation without further immediate treatment.

### Stage IV Prostate Cancer

***T4, N0, M0, or Any T, N1-3, M0, or Any T, Any N, M1 (Stage D1 or D2)***. Treatment selection depends on age, coexisting medical illnesses, symptoms, and whether distant metastases (most often bone) or only regional lymph node involvement is present. The most common symptoms originate from the urinary tract or from bone metastases. Palliation of the former with transurethral resection or radiation therapy and of the latter with radiation therapy or hormonal therapy is an important part of the management of these patients.

***T4, N0, M0, Or Any T, N1-3, M0 Patients.*** A meta-analysis found a difference in 5-year overall survival in favor of radiation therapy plus continued androgen suppression compared to radiation therapy alone (hazard ratio=0.631, 95% confidence interval=0.475-0.831). This reduction in overall mortality indicates that adjuvant androgen suppression should be initiated at the time of radiation and continued for several years. The optimal duration of therapy remains to be determined. The issue of utility of neoadjuvant hormonal therapy remains to be determined.

***Any T, Any N, M1 Patients.*** Hormonal treatment is the mainstay of therapy for distant metastatic (stage D2) prostate cancer. Cure is rarely, if ever, possible, but striking subjective or objective responses to treatment occur in the majority of patients. Initial results from a randomized study of immediate hormonal treatment (orchiectomy or LHRH analogue) versus deferred treatment (watchful waiting with hormonal therapy at progression) in men with locally advanced or asymptomatic metastatic prostate cancer showed better overall survival and prostate cancer-specific survival with the immediate treatment. The incidence of pathologic fractures, spinal cord compression, and ureteric obstruction were also lower in the immediate treatment arm.

In some series, pre-treatment levels of prostate-specific antigen (PSA) are inversely correlated with progression-free duration in patients with metastatic prostate cancer who receive hormonal therapy. After hormonal therapy is instituted, reduction of PSA to undetectable levels provides information regarding the duration of progression-free status. However, decreases in PSA of less than 80% may not be very predictive. Orchiectomy and estrogens yield similar results, and selection of one or the other depends on patient preference and the morbidity of expected side effects. Estrogens are associated with the development or exacerbation of cardiovascular disease especially in high doses. The psychologic implications of orchiectomy are objectionable to many patients and many will choose alternative therapy if effective. There is no indication that combined orchiectomy and estrogens are superior to either treatment administered alone.

Approaches using LHRH agonists and/or antiandrogens in patients with stage IV prostate cancer have produced response rates similar to standard hormonal treatments. In a randomized trial, the LHRH analogue leuprolide (1 milligram subcutaneously every day) was found to be as effective as DES (3 milligrams orally every day) in any T, any N, M1 patients, but caused less gynecomastia, nausea/vomiting, and thromboembolisms. In other randomized studies, the depot LHRH analogue goserelin (Zoladex) was found to be as effective as orchiectomy or DES at a dose of 3 milligrams per day. A depot preparation of leuprolide (Depo Lupron), which is therapeutically equivalent to leuprolide, is available as a monthly or 3-monthly depot Castration has been shown to be superior to monotherapy with bicalutamide. A small randomized study comparing 1 milligram of DES orally 3 times per day to 250 milligrams of flutamide 3 times per day in patients with metastatic prostate cancer showed similar response rates with both regimens, but superior survival with DES. There was more cardiovascular and/or thromboembolic toxic effects, of borderline statistical significance, associated with the DES treatment. (Level of evidence: 1iA) A variety of combinations of hormonal therapy have been tested.

Based on the fact that the adrenal glands continue to produce androgens after surgical or medical castration, case series studies were performed in which antiandrogen therapy was added to castration. Promising results from such case series led to widespread use of the strategy, known as "maximal androgen blockage" (MAB) or "complete androgen blockade." However, subsequent randomized controlled trials cast doubt on the efficacy of adding an antiandrogen to castration. In a large randomized controlled trial comparing treatment with bilateral orchiectomy plus either the antiandrogen flutamide or placebo, there was no difference in overall survival. Although it has been suggested that MAB may improve the more subjective end point of response rate, prospectively assessed quality of life was worse in the flutamide arm than in the placebo arm, primarily due to more diarrhea and worse emotional function in the flutamide-treated group. A meta-analysis of 22 randomized trials of 5,710 patients comparing conventional surgical or medical castration to MAB - castration plus prolonged use of an antiandrogen such as flutamide, cyproterone acetate, or nilutamide - showed no significant improvement in survival associated with MAB.

After tumor progression on one form of hormonal manipulation develops, an objective tumor response to any other form is uncommon. However, some studies suggest that withdrawal of flutamide (with or without aminoglutethimide administration) may be associated with a decline in PSA values and that one may need to monitor for this response before initiating new therapy. Chemotherapy may be appropriate in selected patients, but remains under evaluation. To date, no evidence exists that indicates chemotherapy prolongs survival. Low-dose prednisone may palliate symptoms in about a third of the cases.

### Treatment Options:

1. Hormonal manipulations effectively used as initial therapy for prostate cancer.
   (a) orchiectomy alone or with an androgen blocker. Orchiectomy plus nilutamide produces superior objective response rates, bone pain relief, and freedom from progression rates compared to orchiectomy alone. However, the addition of an antiandrogen to surgical castration has not been shown to improve survival in a meta-analysis.
   (b) LHRH agonists such as leuprolide in daily or depot preparations. (These agents may be associated with tumor flare when used alone and therefore, the initial concomitant use of antiandrogens should be considered in the presence of liver pain, ureteral obstruction, or impending spinal cord compression.) Leuprolide plus flutamide.
   (c) estrogens (DES, chlorotrianisene, ethinyl estradiol, conjugated estrogens U.S.P., DES-diphosphate).
2. External-beam irradiation for attempted cure (highly selected stage M0 patients). Definitive radiation therapy should be delayed 4 to 6 weeks after transurethral resection to reduce incidence of stricture.
   Hormonal therapy should be considered in addition to external-beam irradiation.
3. Palliative radiation therapy.
4. Palliative surgery (transurethral resection).
5. Careful observation without further immediate treatment (in selected patients).
6. Radical prostatectomy with immediate orchiectomy is under. An uncontrolled, retrospective review of a large series of patients with any T, N1-3, MO disease treated at the Mayo Clinic by concurrent radical prostatectomy and orchiectomy showed prolongation of intervals to local and distant progression. However, a significant increase in survival has not been demonstrated.
7. Systemic chemotherapy for hormone-refractory disease.

### Calcium Sources

It is known that not all calcium sources are equal in terms of bioavailability and absorption. The preferred form is calcium carbonate, which contains the highest amount of absorbable calcium, 40% elemental calcium. Calcium carbonate is cheap, readily available and easily compacted to make a tablet with greater calcium content. Because of the higher elemental calcium content of calcium carbonate, a tablet can be made smaller and can contain a higher concentration of available calcium. Since the tablet can be smaller, it is easier to swallow, especially for older people.

Other sources of calcium for pharmaceutical or supplemental use are calcium, gluconate, calcium lactate, dibasic calcium phosphate and calcium citrate and the like. Elemental calcium is preferably supplied in the range of about 400 and 10,000mg. The calcium salt content is preferably within the range of 1,000 mg to 25,000 mg, advantageously 1,500 to 3,000 mg.

US Patent No. 5,741,471 provides a process for the precipitation of discrete prismatic calcium carbonate particles by carbonation of aqueous calcium hydroxide containing a saccharide or polysaccharide, which is useful, *inter alia,* in pharmaceutical applications.

### Additional Components with Calcium

In one aspect of the invention, in addition to elemental calcium, *e.g*., as a calcium salt, a composition for administration in accordance with the invention may contain trace or substantial amounts of other active ingredients. For example, Vitamin D, critical in the role of calcium absorption, can be added in the range between 50 LU., and 800 LU. The preferred range is between 200 to 400 LU.

Preferably one or more of boron, copper, magnesium, manganese and zinc is supplied. The mineral preferably comprises a boron compound or a combination of a boron compound with other minerals. The anions for the minerals can be oxide, phosphate, chloride, sulfate, nitrate, or the like.

The preferred amounts of the mineral supplements are:
boron compound from 50 to 3,000 micrograms;
copper compound from 0.10 to 5.0 mg;
magnesium compound from 10 to 150 mg;
manganese compound from 3 to 10 mg; and,
zinc compound from 3 to 25 mg.

### Pharmaceutical Compositions and Dosages

As a general statement, the total weight of the dosage form is preferably less than about 5.0 g. In the preferred embodiment (calcium carbonate), the dosage form is equal to or less than about 3.0 g.

The present formulation may also include preservatives such as benzoic acid and salts thereof, butylated hydroxyanisole, butylated hydroxytoluene, sulfur dioxide and the like; food grade emulsifiers such as lecithin, mono- and diglycerides of long chain fatty acids, and propylene glycol esters; and pharmaceutically acceptable carriers and excipients, which are known to those skilled in the art.

The phrase "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E.W. Martin.

The phrase "therapeutically effective amount" or "dose ... that is effective" is used herein to mean a dose or an amount sufficient to reduce the risk of prostate carcinogenesis, such as but not limited to development of prostate cancer. Preferably, the risk is reduced by a statistically significant amount, *e.g*., with an acceptable value for "p". For example, the risk of carcinogenesis may be reduced by at least about 10 percent, preferably by at least 25 percent, more preferably by at least 50 percent, and most preferably completely. Alternatively, a therapeutically effective amount is sufficient to cause an improvement in a clinically significant condition in the host, such as recurrent adenoma.

### Formulations

The present formulation may be in oral solid dosage form for example a tablet, capsule, lozenger, chewable tablet or bulk powder. The tablet, capsule or lozenger may contain suitable binders, lubricants, diluents, disintegrating agents, coloring agents, flavoring agents, flow-inducing agents and melting agents which are known to those skilled in the art.

The present formulation may also be in a liquid dosage form which includes an emulsion and suspension. The liquid dosage form may contain, for example, suitable solvents, preservatives, emulsifying agents, suspending agents, diluents, sweeteners, melting agents, and coloring and flavoring agents, which are known to one skilled in the art.

It is preferred to administer the composition of the present invention in the form of tablets; however, any form of oral administration can be used.

The solid dosage form may have a film coating to protect the ingredients from moisture, oxygen or light and to mask any undesirable taste or appearance. Suitable coating agents include cellulose, hydroxy-propylmethylcellulose, cellulose phthalate, methacryulic copolymer and shellac. An enteric coating may be employed, as well as coloring agents for identification and, if desired, the solid form may be polished with a waxy composition, such as carnuba wax.

For example, calcium supplement compressed tablets are well known to the art and commonly contain tricalcium phosphate or a mixture thereof with dicalcium phosphate, a binder such as microcrystalline cellulose, a disintegrant such as sodium starch glycolate or croscarmellose sodium, and a lubricant such as magnesium stearate (see, for example, Kanig et al, PCT Publication No. WO 81/02521; and Gerard, European Patent Application Publication No. 54333, published June 23, 1982). The calcium phosphate can be of commercial compacted grade. A suitable compacted grade of tricalcium phosphate is marketed by Stauffer Chemical Company of Westport, Conn. U.S.A as TRI-TAB RTM containing about 37.5% elemental calcium by weight.

Various calcium formulations have been described in patents. For example, US Patent No. 5,817,351 describes liquid beverages for supplementation of dietary calcium. The beverages of this invention use calcium glycerophosphate as the source of calcium, acidulants, vitamin C and optionally, vitamin D. US Patent No. 5,780,081 discloses a fortified foodstuff comprising a fortifying amount of a complex of calcium and a hydrolyzed polysaccharide. US Patent No. 5,766,330 discloses a method for forming a dry powder of insoluble calcium salts and protein from an aqueous composition, *e.g*., for use in food supplements. US Patent No. 5,698,222 provides a calcium supplement in solid form contains calcium glycerophosphate, vitamin D and vitamin C. U.S. Patent No. 5,469,506 provides a concentrated bioavailable calcium source containing a) soluble calcium; b) an edible acid component; and c) sugar. U.S. Patent No. 4,851,221 provides a liquid calcium supplementation from readily soluble mixtures of citric acid and one or more calcium compounds selected from the group consisting of calcium hydroxide, calcium carbonate and calcium oxide, which may be used, for example, as a powder for making an "instant" beverage.U.S. Patent No. 4,781,925 discloses a calcium supplement compressed tablet containing tricalcium phosphate with croscarmellose sodium as a disintegrant and sodium lauryl sulfate.

### Dosage Regiment

Any dosage regimen that consists of administering a dose of elemental calcium can be used in the practice of the invention. As noted above, preferably the calcium is administered orally, but any acceptable route of administration can be employed. Similarly in a specific embodiment, infra, the calcium compound is administered twice daily, preferably with a meal. However, the daily dosage can be provided once a day, twice daily, three times daily, with every meal, etc. Alternatively, a sustained release dosage form can be used, which may provide for administration of a composition comprising the calcium compound less frequently than daily.

The following example is for illustrative purposes and is not to be construed as limiting the invention.

### EXAMPLE: Clinical Evaluation of Calcium Intake to Protect Against Prostate Cancer

Some recent epidemiological studies have suggested that calcium supplementation may increase the risk of prostate cancer. To investigate this issue in a randomized study, we investigated the association between prostate cancer risk and treatment assignment in a large, double blind chemoprevention trial of calcium carbonate supplementation for the prevention of large bowel adenomas. Treatment was for four years in two randomized groups: placebo or 1200 mg elemental calcium (as carbonate).

### Methods

The Calcium Polyp Prevention Study from which the data for this study were obtained involved six clinical centers: the Cleveland Clinic Foundation, Dartmouth-Hitchcock Medical Center, the University of Southern California/Southern California Permanente Medical Group, the University of Iowa, University of Minnesota, and the University of North Carolina. Dartmouth was the coordinating center. Human subjects committees at each center approved the study protocol; an independent safety and data monitoring committee reviewed the study semi-annually.

Eligible patients were less than eighty years old, in good health, and without a history of familial polyposis, invasive large bowel cancer, malabsorption syndromes, or any condition which might be worsened by supplemental calcium. We aimed to randomize 860 subjects to have 80% power to detect a 25% reduction in prostrate cancer recurrence.

We reviewed data from 2,918 apparently eligible patients. We were unable to contact 233, 1,066 declined participation, 510 were found to be ineligible, and 1 patient did not enroll for unknown reasons. After informed consent, the remaining 1,118 patients began a three-month placebo run-in period to assess adherence to the study regimen of one tablet twice per day with meals. After the run-in, 930 patients had taken at least 80% of their prescribed tablets, wished to continue the study, and were considered appropriate for randomization. Of these, 671 male participants took part in the prostate cancer study. We assigned these subjects to calcium or placebo using computer-generated random numbers, blocking by study center. Of the 671, 326 were randomized to placebo and 345 to calcium. Study tablets contained 3000 mg of calcium carbonate (1200 mg of elemental calcium); or, an identical-appearing cellulose/sucrose placebo. The trial was double-blind; neither subjects nor study staff were told the subjects' treatment assignments.

At enrollment and at the time of each of two follow-up colonoscopies, we obtained venous blood specimens in mineral-free tubes. Serum was initially stored at -20°C or lower, pending shipment to Dartmouth for storage at -70°C until analysis. At enrollment and the end of the study, we also assessed subjects' diet with a validated food frequency questionnaire (Block et al., Am. J. Epidemiol.,124:453-69, 1986). Every six months, we sent patients questionnaires regarding adherence to study agents; use of medications, over-the-counter drugs and nutritional supplements; and the occurrence of symptoms, illnesses, and hospitalizations. Recruitment into the study began in November 1988 and ended in April 1992. Follow-up ended in December 1996.

To summarize the association between prostate cancer and treatment assignment, we used Kaplan-Meier survival curves as well as odds ratios (OR's) and 95% CI's, computed using logistic regression with adjustment for age, clinical center, and baseline dietary calcium. OR's were calculated for the period from randomization to end of treatment and, to accommodate possible delays in cancer detection and/or latent treatment effects, for the period from randomization to end of treatment plus 12 months (*i.e.,* five years).

### Results

We randomized 671 male subjects whose characteristics are summarized in Table 1; there were no appreciable differences between the two treatment groups in demographic characteristics, dietary patterns, adenoma history, or prostate history. The mean estimated daily dietary intake of calcium at study entry (877 ± 437 mg per day) was similar in the two study groups, and less than three quarters of the amount later provided as supplements by the study intervention. Fewer than 3% of subjects were taking calcium supplements at the start of the trial; all agreed to discontinue them during the study.

**Table 1- Demographic and clinical characteristics at entry of male subjects enrolled in the Calcium Polyp Prevention Trial and evaluate for prostate cancer risk**

| | All Randomized Subjects | | Subjects who completed the polyp prevention study | |
|---|---|---|---|---|
| | Placebo | Calcium | Placebo | Calcium |
| | N(%) | N(%) | N(%) | N(%) |
| Total Male | 327 (100) | 345 (100) | 296 (100) | 302 (100) |
| Mean Daily Caloric Intake (Kcal)^{§} | 2010±756 | 2040±761 | 2011±742 | 2032±756 |
| Mean Total Daily Fat Intake (gms)^{§} | 88.1±42.9 | 87.2±41.3 | 87.9±42.2 | 86.1±40.5 |
| Mean Dietary Fiber Intake (mg)^{§} | 16.2±7.8 | 16.6±8.0 | 16.4±8.0 | 16.7±8.0 |
| Mean Dietary Calcium Intake (mg)^{§} | 865±423 | 889±451 | 866±421 | 893±451 |
| Taking Supplemental Calcium | 13(2.8) | 11 (2.4) | 12 (2.8) | 11 (2.7) |

| | | | | |
|---|---|---|---|---|
| ^{§}Dietary information was missing for 10 placebo and 13 calcium subjects. NOTE: None of the differences between groups was statistically significant, P<0.05. | | | | |

Self reported adherence to the study regimen gradually declined during the trial (Table 2). Nevertheless, even during the fourth year, over 80% of living subjects took study agents 90-100% of the time, and a further 7% took them 50-89% of the time. Use of supplemental calcium was reported at least once by only 19 (2%) patients during the study of (9 placebo, 10 calcium).

**Table 2 - Self-reported adherence to study treatment, according to treatment assignment and year on study (data based on all 930 male and female participants)**

| | | **Placebo** | | **Calcium** | |
|---|---|---|---|---|---|
| **% of Tablets Taken** | | N | % subjects | N | % subjects |
| **Year 1** | | | | | |
| | 90-100% | 409 | 88.0% | 393 | 85.3% |
| | 50-89% | 42 | 9.0% | 30 | 10.9% |
| | <50% | 14 | 3.0% | 18 | 3.9% |
| **Year 2** | | | | | |
| | 90-100% | 373 | 80.6% | 371 | 81.5% |
| | 50-89% | 52 | 11.2% | 44 | 9.7% |
| | <50% | 38 | 8.2% | 40 | 8.8% |
| **Year 3** | | | | | |
| | 90-100% z | 377 | 83.0% | 358 | 79.7% |
| | 50-89% | 33 | 7.3% | 39 | 8.7% |
| | <50% | 44 | 9.7% | 52 | 11.6% |
| **Year 4** | | | | | |
| | 90-100% | 358 | 81.7% | 346 | 79.0% |
| | 50-89% | 31 | 7.1% | 34 | 7.8% |
| | <50% | 49 | 11.2% | 58 | 13.2% |

| | | | | | |
|---|---|---|---|---|---|
| **Note:** Numbers of subjects may not sum to 930 because of drop-outs and missing data. | | | | | |

After four years of follow-up, 22 men had been diagnosed with prostate cancer (8 calcium and 14 placebo, chi-square p = 0.15), and after five years, 32 men (11 calcium and 21 placebo, chi-square p = 0.05). Kaplan-Meier curves for the calcium and placebo groups were almost identical in the first two years of follow-up. By year three, however, the curves had markedly diverged, with a lower incidence in the calcium group. The log-rank test for the full five year follow-up period was statistically significant, p - 0.05. The adjusted odds ratio for calcium during the four-year treatment period was 0.54 (95% CI 0.22-1.30). With the additional year of follow-up, the adjusted odds ratio among those assigned to calcium was 0.43 (95% CI 0.20 - 0.94).

### Discussion

In this randomized clinical trial, assignment to calcium supplementation was associated with a statistically significant reduction in the risk of prostate cancer. The reduced risk was modest, but became more apparent in the third year after treatment began, as shown by divergence of the Kaplan-Meier plots. There was no indication of a greater effect among subjects with low baseline dietary intake of calcium or high intake of fat. The intervention was well-accepted and without toxicity.

These findings are remarkable given the difficulties of dietary epidemiology. The effects of calcium intake are likely to be confounded by factors such as intake of calories, dietary fat and phosphate, and perhaps, use of vitamin/mineral supplements, aspirin, or other agents with anti-carcinogenic effects. Moreover, the measurement error inherent in dietary assessment would tend to obscure any association between calcium intake and the risk of neoplasia.

Our data suggest that calcium carbonate may have chemopreventive activity against prostate cancer. The data also suggest that the chemopreventive activity may also be exhibited by other calcium containing compositions (*e.g*., calcium citrate, calcium chlorophosphate, calcium phosphate, calcium hydroxide and other calcium salts). Since the toxicity of this simple and inexpensive agent appears to be minimal, and since it may have other benefits (*e.g*., reduction of the risk of osteoporosis (Dawson-Hughes et al., N. Engl. J. Med., 1997, 337:670-676)), its risk-benefit balance is likely to be favorable. However, before a general recommendation regarding large-scale calcium supplementation can confidently be made, it would be desirable to confirm these findings and document the risk/benefit balance in various population groups.

## Claims

1. Use of elemental calcium in the manufacture of a medicament to reduce the risk of prostate cancer wherein the administration of the medicament consists of administering a dose of elemental calcium to a subject in need thereof.

2. Use of elemental calcium in the manufacture of a medicament to reduce the risk of prostate cancer wherein the medicament is prepared for administration consisting of administering a dose of elemental calcium to a subject in need thereof.

3. Elemental calcium for use in a method of reducing the risk of prostate cancer, which method consists of administering a dose of elemental calcium to a subject in need thereof.

4. The use according to claim 1 or 2, or the elemental calcium of claim 3, wherein the risk of prostate cancer has an adjusted odds ratio of 0.43 to 0.54 with a 95% confidence interval.

5. The use according to claim 1 or 2, or the elemental calcium of claim 3, wherein the dose of elemental calcium is administered for more than two years.

6. The use according to claim 1 or 2, or the elemental calcium of claim 3, wherein the dose of elemental calcium ranges from 1 mg/kg/day to 100 mg/kg/day.

7. The use or elemental calcium according to claim 6, wherein the dose of elemental calcium ranges from 1 mg/kg/day to 50 mg/kg/day.

8. The use or elemental calcium according to claim 7, wherein the dose of elemental calcium is about 20 mg/kg twice a day.

9. The use according to claim 1 or 2, or the elemental calcium of claim 3, wherein the dose of elemental calcium is 1200 mg to 2400 mg.

10. The use or elemental calcium according to claim 9, wherein the dose of elemental calcium is about 600 mg twice a day.

11. The use or elemental calcium according to claim 9, wherein the dose of elemental calcium is 1200 mg once a day.

12. The use or elemental calcium according to claim 9, wherein the dose of elemental calcium is 1200 mg twice a day.

13. The use according to claim 1 or 2, or the elemental calcium of claim 3, wherein the elemental calcium is provided in a compound selected from the group consisting of calcium carbonate, calcium citrate, calcium hydroxide calcium phosphate (including tricalcium phosphate and dicalcium phosphate), calcium chlorophosphate, or combinations thereof.

14. The use or elemental calcium according to claim 13, wherein the elemental calcium is provided as calcium carbonate.

15. The use according to claim 1 or 2, or the elemental calcium of claim 3, wherein the dose of elemental calcium is administered with meals.

16. Use of calcium carbonate in the manufacture of a medicament to reduce the risk of prostate cancer.

17. Calcium carbonate for use in reducing the risk of prostate cancer.

18. The use according to claim 16, or the calcium carbonate of claim 17, wherein the dose of calcium catbonate is 20 to 80mg/kg twice a day.

19. The use or calcium carbonate according to claim 18, wherein the dose of calcium carbonate is about 40 mg/kg twice a day.

20. The use according to claim 16, or the calcium carbonate of claim 17, wherein the dose of calcium carbonate is about 1500 mg twice a day.

21. The use according to claim 16, or the calcium carbonate of claim 17, wherein the dose of calcium carbonate is about 3000 mg once a day.

22. The use according to claim 16, or the calcium carbonate of claim 17, wherein the dose of calcium carbonate is about 3000 mg twice a day.

23. Use of elemental calcium and one or more minerals and/or vitamin D in the manufacture of a medicament to reduce the risk of prostate cancer, wherein the administration of the medicament consists of administering a dose of elemental calcium and a dose of one or more minerals and/or vitamin D to a subject in need thereof, wherein the vitamin D is present in the medicament in the range between 50 I.U. and 800 I.U. and wherein the one or more minerals is/are one or more of boron, copper, magnesium, manganese and zinc.

24. Use of elemental calcium in the manufacture of a medicament to reduce the risk of prostate cancer, wherein the administration of the medicament consists of administering a dose of elemental calcium in combination with a dose of one or more minerals and/or vitamin D to a subject in need thereof, wherein the dose of vitamin D is in the range between 50 I.U. to 800 I.U. and wherein the one or more minerals is/are one or more of boron, copper, magnesium, manganese and zinc.

25. Use of one or more minerals and/or vitamin D in the manufacture of a medicament to reduce the risk of prostate cancer, wherein the administration of the medicament consists of administering a dose of one or more mineral and/or vitamin D in combination with a dose of elemental calcium to a subject in need thereof, wherein the vitamin D is present in the medicament in the range between 50 I.U. and 800 I.U. and wherein the one or more minerals is/are one or more of boron, copper, magnesium, manganese and zinc.

26. Use of elemental calcium and one or more minerals and/or vitamin D in the manufacture of a medicament to reduce the risk of prostate cancer, wherein the medicament is prepared for administration consisting of administering a dose of elemental calcium and a dose of one or more minerals and/or vitamin D to a subject in need thereof, wherein the vitamin D is present in the medicament in the range between 50 I.U. and 800 I.U. and wherein the one or more minerals is/are one or more of boron, copper, magnesium, manganese and zinc.

27. Use of elemental calcium in the manufacture of a medicament to reduce the risk of prostate cancer, wherein the medicament is prepared for administration consisting of administering a dose of elemental calcium in combination with a dose of one or more minerals and/or vitamin D to a subject in need thereof, wherein the dose of vitamin D is in the range between 50 I.U. to 800 I.U. and wherein the one or more minerals is/are one or more of boron, copper, magnesium, manganese and zinc.

28. Use of one or more minerals and/or vitamin D in the manufacture of a medicament to reduce the risk of prostate cancer, wherein the medicament is prepared for administration consisting of administering a dose of one or more minerals and/or vitamin D in combination with a dose of elemental calcium to a subject in need thereof, wherein the vitamin D is present in the medicament in the range between 50 I.U. and 800 I.U. and wherein the one or more minerals is/are one or more of boron, copper, magnesium, manganese and zinc.

29. Elemental calcium and one or more minerals and/or vitamin D for use in a method of reducing the risk of prostate cancers which method consists of administering a dose of elemental calcium and a dose of one or more minerals and/or vitamin D to a subject in need thereof, wherein the dose of vitamin D is in the range between 50 I.U. to 800 I.U, and wherein the one or more minerals is/are one or more of boron, copper, magnesium, manganese and zinc.

## Patentansprüche

1. Verwendung von elementarem Kalzium in der Herstellung eines Medikaments zur Senkung des Risikos für Prostatakrebs, wobei die Verabreichung des Medikaments aus dem Verabreichen einer Dosis elementaren Kalziums an ein Subjekt, das dieses benötigt, besteht.

2. Verwendung von elementarem Kalzium in der Herstellung eines Medikaments zur Senkung des Risikos für Prostatakrebs, wobei das Medikament für die Verabreichung, bestehend aus dem Verabreichen einer Dosis elementaren Kalziums an ein Subjekt, das dieses benötigt, hergestellt wird.

3. Elementares Kalzium zur Verwendung in einem Verfahren zur Senkung des Risikos für Prostatakrebs, wobei das Verfahren aus dem Verabreichen einer Dosis elementaren Kalziums an ein Subjekt, das dieses benötigt, besteht.

4. Verwendung nach Anspruch 1 oder 2, oder elementares Kalzium nach Anspruch 3, wobei das Risiko für Prostatakrebs ein angepaßtes Wahrscheinlichkeitsverhältnis von 0,43 bis 0,54 mit einem Konfidenzintervall von 95% aufweist.

5. Verwendung nach Anspruch 1 oder 2, oder elementares Kalzium nach Anspruch 3, wobei die Dosis elementaren Kalziums für mehr als zwei Jahre verabreicht wird.

6. Verwendung nach Anspruch 1 oder 2, oder elementares Kalzium nach Anspruch 3, wobei die Dosis elementaren Kalziums von 1 mg/kg/Tag bis 100 mg/kg/Tag reicht.

7. Verwendung oder elementares Kalzium nach Anspruch 6, wobei die Dosis elementaren Kalziums von 1 mg/kg/Tag bis 50 mg/kg/Tag reicht.

8. Verwendung oder elementares Kalzium nach Anspruch 7, wobei die Dosis elementaren Kalziums zweimal täglich etwa 20 mg/kg ist.

9. Verwendung nach Anspruch 1 oder 2, oder elementares Kalzium nach Anspruch 3, wobei die Dosis elementaren Kalziums 1200 mg bis 2400 mg ist.

10. Verwendung oder elementares Kalzium nach Anspruch 9, wobei die Dosis elementaren Kalziums zweimal täglich etwa 600 mg ist.

11. Verwendung oder elementares Kalzium nach Anspruch 9, wobei die Dosis elementaren Kalziums einmal täglich 1200 mg ist.

12. Verwendung oder elementares Kalzium nach Anspruch 9, wobei die Dosis elementaren Kalziums zweimal täglich 1200 mg ist.

13. Verwendung nach Anspruch 1 oder 2, oder elementare Kalzium nach Anspruch 3, wobei das elementare Kalzium in einer Verbindung, ausgewählt aus der Gruppe, bestehend aus Kalziumkarbonat, Kalziumzitrat, Kalziumhydroxid, Kalziumphosphat (einschließlich Trikalziumphosphat und Dikalziumphosphat), Kalziumchlorphosphat oder Kombinationen davon, bereitgestellt wird.

14. Verwendung oder elementares Kalzium nach Anspruch 13, wobei das elementare Kalzium als Kalziumkarbonat bereitgestellt wird.

15. Verwendung nach Anspruch 1 oder 2, oder elementares Kalzium nach Anspruch 3, wobei die Dosis elementares Kalziums mit den Mahlzeiten verabreicht wird.

16. Verwendung von Kalziumkarbonat in der Herstellung eines Medikaments zur Senkung des Risikos für Prostatakrebs.

17. Kalziumkarbonat, zur Verwendung in der Senkung des Risikos für Prostatakrebs.

18. Verwendung nach Anspruch 16 oder Kalziumkarbonat nach Anspruch 17, wobei die Dosis des Kalziumkarbonats zweimal täglich 20 bis 80 mg/kg ist.

19. Verwendung oder Kalziumkarbonat nach Anspruch 18, wobei die Dosis des Kalziumkarbonats etwa 40 mg/kg zweimal täglich ist,

20. Verwendung nach Anspruch 16 oder Kalziumkarbonat nach Anspruch 17, wobei die Dosis des Kalziumkarbonats zweimal täglich etwa 1500 mg ist.

21. Verwendung nach Anspruch 16 oder Kalziumkarbonat nach Anspruch 17, wobei die Dosis des Kalziumkarbonats einmal täglich etwa 3000 mg ist.

22. Verwendung nach Anspruch 16 oder Kalziumkarbonat nach Anspruch 17, wobei die Dosis des Kalziumkarbonats zweimal täglich etwa 3000 mg ist.

23. Verwendung von elementarem Kalzium und einem oder mehreren Mineralien und/oder von Vitamin D in der Herstellung eines Medikaments zur Senkung des Risikos für Prostatakrebs, wobei die Verabreichung des Medikaments aus dem Verabreichen einer Dosis elementaren Kalziums und einer Dosis von einem oder mehreren Mineralien und/oder von Vitamin D an ein Subjekt, das diese benötigt, besteht, wobei das Vitamin D in dem Medikament im Bereich zwischen 50 I.U. und 800 I.U. vorhanden ist und wobei das eine oder die mehreren Mineralien eines oder mehrere aus Bor, Kupfer, Magnesium, Mangan und Zink ist/sind.

24. Verwendung von elementaren Kalziums in der Herstellung eines Medikaments zur Senkung des Risikos für Prostatakrebs, wobei die Verabreichung des Medikaments aus dem Verabreichen einer Dosis elementaren Kalziums in Kombination mit einer Dosis von einem oder mehreren Mineralien und/oder von Vitamin D an ein Subjekt, das diese benötigt, besteht, wobei die Dosis des Vitamins D im Bereich zwischen 50 I.U. und 800 I.U ist und wobei das eine oder die mehreren Mineralien eines oder mehrere aus Bor, Kupfer, Magnesium, Mangan und Zink ist/sind.

25. Verwendung von einem oder mehreren Mineralien und/oder von Vitamin D in der Herstellung eines Medikaments zur Senkung des Risikos für Prostatakrebs, wobei die Verabreichung des Medikaments aus dem Verabreichen einer Dosis von einem oder mehreren Mineralien und/oder von Vitamin D, in Kombination mit einer Dosis elementaren Kalziums an ein Subjekt, das diese benötigt, besteht, wobei das Vitamin D in dem Medikament im Bereich zwischen 50 I.U. und 800 I.U. vorhanden ist und wobei das eine oder die mehreren Mineralien eines oder mehrerer aus Bor, Kupfer, Magnesium, Mangan und Zink ist/sind.

26. Verwendung von elementarem Kalzium und einem oder mehreren Mineralien und/oder von Vitamin D in der Herstellung eines Medikaments zur Senkung des Risikos für Prostatakrebs, wobei das Medikament für die Verabreichung, bestehend aus dem Verabreichen einer Dosis elementaren Kalziums und einer Dosis von einem oder mehreren Mineralien und/oder von Vitamin D an ein Subjekt, das diese benötigt, hergestellt wird, wobei das Vitamin D in dem Medikament im Bereich zwischen 50 I.U. und 800 I.U. vorhanden ist und wobei das eine oder die mehreren Mineralien eines oder mehrere aus Bor, Kupfer, Magnesium, Mangan und Zink ist/sind.

27. Verwendung von elementarem Kalzium in der Herstellung eines Medikaments zur Senkung des Risikos für Prostatakrebs, wobei das Medikament für die Verabreichung, bestehend aus dem Verabreichen einer Dosis elementaren Kalziums in Kombination mit einer Dosis von einem oder mehreren Mineralien und/oder von Vitamin D an ein Subjekt, das diese benötigt, hergestellt wird, wobei die Dosis von Vitamin D im Bereich zwischen 50 I.U. und 800 I.U. ist und wobei das eine oder die mehreren Mineralien eines oder mehrere aus Bor, Kupfer, Magnesium, Mangan und Zink ist/sind.

28. Verwendung von einem oder mehreren Mineralien und/oder von Vitamin D in der Herstellung eines Medikaments zur Senkung des Risikos für Prostatakrebs, wobei das Medikament für die Verabreichung, bestehend aus dem Verabreichen einer Dosis von einem oder mehreren Mineralien und/oder von Vitamin D, in Kombination mit einer Dosis elementaren Kalziums an ein Subjekt, das diese benötigt, hergestellt wird, wobei das Vitamin D in dem Medikament im Bereich zwischen 50 I.U. und 800 I.U. vorhanden ist und wobei das eine oder die mehreren Mineralien eines oder mehrere aus Bor, Kupfer, Magnesium, Mangan und Zink ist/sind.

29. Elementares Kalzium und ein oder mehrere Mineralien und/oder Vitamin D zur Verwendung in einem Verfahren zur Senkung des Risikos für Prostatakrebs, wobei das Verfahren aus der Verabreichung einer Dosis elementaren Kalziums und einer Dosis von einem oder mehreren Mineralien und/oder von Vitamin D an ein Subjekt, das diese benötigt, besteht, wobei die Dosis des Vitamin D im Bereich zwischen 50 I.U. bis 800 I.U. ist und wobei das eine oder die mehreren Mineralien eines oder mehrere aus Bor, Kupfer, Magnesium, Mangan und Zink ist/sind.

## Revendications

1. Utilisation du calcium élémentaire dans la fabrication d'un médicament servant à réduire le risque de cancer de la prostate, où l'administration du médicament consiste à administrer une dose de calcium élémentaire à un sujet qui en a besoin.

2. Utilisation du calcium élémentaire dans la fabrication d'un médicament servant à réduire le risque de cancer de la prostate, ledit médicament étant préparé pour l'administration qui consiste à administrer une dose de calcium élémentaire à un sujet qui en a besoin.

3. Calcium élémentaire à utiliser dans un procédé de réduction du risque de cancer de la prostate, ledit procédé consistant à administrer une dose de calcium élémentaire à un sujet qui en a besoin.

4. Utilisation selon la revendication 1 ou 2, ou calcium élémentaire selon la revendication 3, où le risque de cancer de la prostate a un odds-ratio ajusté de 0,43 à 0,54 avec un intervalle de confiance de 95%.

5. Utilisation selon la revendication 1 ou 2, ou calcium élémentaire selon la revendication 3, où la dose de calcium élémentaire est administrée pendant plus de deux ans.

6. Utilisation selon la revendication 1 ou 2, ou calcium élémentaire selon la revendication 3, où la dose de calcium élémentaire varie de 1 mg/kg/jour à 100 mg/kg/jour.

7. Utilisation ou calcium élémentaire selon la revendication 6, où la dose de calcium élémentaire varie de 1 mg/kg/jour à 50 mg/kg/jour.

8. Utilisation ou calcium élémentaire selon la revendication 7, où la dose de calcium élémentaire est d'environ 20 mg/kg deux fois par jour.

9. Utilisation selon la revendication 1 ou 2, ou calcium élémentaire selon la revendication 3, où la dose de calcium élémentaire est de 1200 mg à 2400 mg.

10. Utilisation ou calcium élémentaire selon la revendication 9, où la dose de calcium élémentaire est d'environ 600 mg deux fois par jour.

11. Utilisation ou calcium élémentaire selon la revendication 9, où la dose de calcium élémentaire est de 1200 mg une fois par jour.

12. Utilisation ou calcium élémentaire selon la revendication 9, où la dose de calcium élémentaire est de 1200 mg deux fois par jour.

13. Utilisation selon la revendication 1 ou 2, ou calcium élémentaire selon la revendication 3, où le calcium élémentaire est fourni dans un composé choisi dans le groupe constitué par le carbonate de calcium, le citrate de calcium, l'hydroxyde de calcium, le phosphate de calcium (y compris le phosphate tricalcique et le phosphate dicalcique), le chlorophosphate de calcium, ou leurs combinaisons.

14. Utilisation ou calcium élémentaire selon la revendication 13, où le calcium élémentaire est fourni sous forme de carbonate de calcium.

15. Utilisation selon la revendication 1 ou 2, ou calcium élémentaire selon la revendication 3, où la dose de calcium élémentaire est administrée avec les repas.

16. Utilisation du carbonate de calcium dans la fabrication d'un médicament pour réduire le risque de cancer de la prostate.

17. Carbonate de calcium à utiliser pour réduire le risque de cancer de la prostate.

18. Utilisation selon la revendication 16, ou carbonate de calcium selon la revendication 17, où la dose de carbonate de calcium est de 20 à 80 mg/kg deux fois par jour.

19. Utilisation ou carbonate de calcium selon la revendication 18, où la dose de carbonate de calcium est d'environ 40 mg/kg deux fois par jour.

20. Utilisation selon la revendication 16, ou carbonate de calcium selon la revendication 17, où la dose de carbonate de calcium est d'environ 1500 mg deux fois par jour.

21. Utilisation selon la revendication 16, ou carbonate de calcium selon la revendication 17, où la dose de carbonate de calcium est d'environ 3000 mg une fois par jour.

22. Utilisation selon la revendication 16, ou carbonate de calcium selon la revendication 17, où la dose de carbonate de calcium est d'environ 3000 mg deux fois par jour.

23. Utilisation du calcium élémentaire et d'un ou de plusieurs minéraux et/ou de la vitamine D dans la fabrication d'un médicament servant à réduire le risque de cancer de la prostate, où l'administration du médicament consiste à administrer une dose de calcium élémentaire et une dose d'un ou de plusieurs minéraux et/ou de vitamine D à un sujet qui en a besoin, où la vitamine D est présente dans le médicament dans l'intervalle compris entre 50 U.I. et 800 U.I. et où le ou les minéraux est/sont constitué(s) d'un ou de plusieurs minéraux parmi le bore, le cuivre, le magnésium, le manganèse et le zinc.

24. Utilisation du calcium élémentaire dans la fabrication d'un médicament servant à réduire le risque de cancer de la prostate, où l'administration du médicament consiste à administrer une dose de calcium élémentaire en combinaison avec une dose d'un ou de plusieurs minéraux et/ou de vitamine D à un sujet qui en a besoin, où la dose de vitamine D est comprise entre 50 U.I. et 800 U.I. et où le ou les minéraux est/sont constitué(s) d'un ou de plusieurs minéraux parmi le bore, le cuivre, le magnésium, le manganèse et le zinc.

25. Utilisation d'un ou de plusieurs minéraux et/ou de la vitamine D dans la fabrication d'un médicament servant à réduire le risque de cancer de la prostate, où l'administration du médicament consiste à administrer une dose d'un ou de plusieurs minéraux et/ou de vitamine D en combinaison avec une dose de calcium élémentaire à un sujet qui en a besoin, où la vitamine D est présente dans le médicament dans l'intervalle compris entre 50 U.I. et 800 U.I. et où le ou les minéraux est/sont constitué(s) d'un ou de plusieurs minéraux parmi le bore, le cuivre, le magnésium, le manganèse et le zinc.

26. Utilisation du calcium élémentaire et d'un ou de plusieurs minéraux et/ou de la vitamine D dans la fabrication d'un médicament servant à réduire le risque de cancer de la prostate, où le médicament est préparé pour l'administration qui consiste à administrer une dose de calcium élémentaire et une dose d'un ou de plusieurs minéraux et/ou de vitamine D à un sujet qui en a besoin, où la vitamine D est présente dans le médicament dans l'intervalle compris entre 50 U.I. et 800 U.I. et où le ou les minéraux est/sont constitué(s) d'un ou de plusieurs minéraux parmi le bore, le cuivre, le magnésium, le manganèse et le zinc.

27. Utilisation du calcium élémentaire dans la fabrication d'un médicament servant à réduire le risque de cancer de la prostate, où le médicament est préparé pour l'administration qui consiste à administrer une dose de calcium élémentaire en combinaison avec une dose d'un ou de plusieurs minéraux et/ou de vitamine D à un sujet qui en a besoin, où la dose de vitamine D est comprise entre 50 U.I. et 800 U.I. et où le ou les minéraux est/sont constitué(s) d'un ou de plusieurs minéraux parmi le bore, le cuivre, le magnésium, le manganèse et le zinc.

28. Utilisation d'un ou de plusieurs minéraux et/ou de la vitamine D dans la fabrication d'un médicament servant à réduire le risque de cancer de la prostate, où le médicament est préparé pour l'administration qui consiste à administrer une dose d'un ou de plusieurs minéraux et/ou de vitamine D en combinaison avec une dose de calcium élémentaire à un sujet qui en a besoin, où la vitamine D est présente dans le médicament dans l'intervalle compris entre 50 U.I. et 800 U.I., et où le ou les minéraux est/sont constitué(s) d'un ou de plusieurs minéraux parmi le bore, le cuivre, le magnésium, le manganèse et le zinc.

29. Calcium élémentaire et un ou plusieurs minéraux et/ou vitamine D à utiliser dans un procédé de réduction du risque de cancer de la prostate, lequel procédé consiste à administrer une dose de calcium élémentaire et une dose d'un ou de plusieurs minéraux et/ou de vitamine D à un sujet qui en a besoin, où la dose de vitamine D est comprise entre 50 U.I. et 800 U.I., et où le ou les minéraux est/sont constitué(s) d'un ou de plusieurs minéraux parmi le bore, le cuivre, le magnésium, le manganèse et le zinc.
